# EUROPEAN PATENT SPECIFICATION

(11) **EP 2 318 131 B1**
(45) Date of publication and mention of the grant of the patent: **12.08.2015**
(21) Application number: 09847668.2
(22) Date of filing: 04.09.2009
(51) Int. Cl.: B01J 23/70

(54) **CATALYST FOR DIRECT PRODUCTION OF LIGHT OLEFINS AND PREPARATION METHOD THEREOF**
KATALYSATOR ZUR DIREKTHERSTELLUNG LEICHTER OLEFINE UND VERFAHREN ZU SEINER HERSTELLUNG
CATALYSEUR POUR LA PRODUCTION DIRECTE D'OLÉFINES LÉGÈRES ET SON PROCÉDÉ DE PRÉPARATION

(43) Date of publication of application: 11.05.2011
(73) Proprietor: Korea Research Institute of Chemical Technology, Yuseong-gu, Daejeon 305-600 (KR)
(72) Inventor: KANG, Suk-Hwan, Yuseong-gu Daejeon 305-752 (KR); BAE, Jong-Wook, Yuseong-gu Daejeon 305-345 (KR); JUN, Ki-won, Yuseong-gu Daejeon 305-761 (KR); LEE, Yun-Jo, Yuseong-gu Daejeon 305-761 (KR); KIM, Hyo Jean, Yuseong-gu Daejeon 305-804 (KR)
(74) Representative: Charrier, Rapp & Liebau
(86) International application number: PCT/KR2009/004998
(87) International publication number: WO 2011/027921

(56) References cited:
- WO-A2-2010/143783
- CN-A- 1 446 884
- CN-A- 101 049 568
- KR-B1- 100 933 062
- US-A- 4 166 101
- US-A- 4 380 589
- TAO Z ET AL: "Effect of calcium promoter on a precipitated iron-manganese catalyst for Fischer-Tropsch synthesis", CATALYSIS COMMUNICATIONS, ELSEVIER SCIENCE, AMSTERDAM, NL, vol. 7, no. 12, 1 December 2006 (2006-12-01), pages 1061-1066, XP024973129, ISSN: 1566-7367, DOI: 10.1016/J.CATCOM.2006.05.009 [retrieved on 2006-12-01]

## Description

### [Technical Field]

The present invention relates to a method for preparing a Fischer-Tropsch (F-T hereinafter) catalyst system suitable for the selective production of light olefins from syngas produced from natural gas (syngas to olefins; STO), which contains an iron-based catalyst and a calcium oxide (CaOx) prepared by a sol-gel method and shows an increased yield of C₂-C₄ light olefins and improved catalyst stability.

### [Background Art]

In the publication of TAO Z et al "effect of calcium promoter on a precipitated iron-manganese catalyst for Fischer-Tropsch synthesis", in CATALYSIS COMMUNICATIONS, Elsevier Science, Amsterdam NL, vol. 7, no. 12, published on December 1, 2006 on pages 1061-1066, the preparation of calcium promoted precipitated iron-manganese catalysts for Fischer-Tropsch synthesis are disclosed. The catalysts are prepared by the combination of co-precipitated and spray dried method. In published Chinese patent application CN 101 049 068-A the preparation of effused Fe-Co catalyst for the Fischer-Tropsch synthesis with a co-catalyst consisting of K₂O Al₂O₃, CaO and Co is disclosed.

Technologies for converting natural gas to liquid hydrocarbons begin with the gasification of coal or biomass and reforming of natural gas to synthesis gas (syngas). Generally, the F-T reaction transforms syngas to hydrocarbons, and the general chemistry of the F-T reaction is as follows:

nCO+ (2n+1) H₂ → CₙH₂ₙ₊₂+nH₂O (1)

CO+H₂O → CO₂+H₂ (2)

2 nCO+ (n+1) H₂ → CₙH₂ₙ₊₂+nCO₂ (3)

In the water-gas shift reaction (equation (2) competing with the STO reaction, the carbon monoxide reacts with the water generated from equation (1), to form carbon dioxide and hydrogen. Thus, the water produced in equation (1) alters the ratio of hydrogen to carbon monoxide in the product of the F-T process.

Catalysts used in the F-T process vary in composition based upon the product mixture desired and reaction conditions employed but commonly comprise: at least one active metal selected from the elements of the group VIIIA of the Periodic Table, preferably Co, Ru, Fe or Ni; optionally, at least one promoter selected from the elements of Groups IIIA, IVA, VA, VIA or the like; and optionally, at least one promoter selected from Group IB (U.S. Patent No. 7067562 B2).

The active metal used in the F-T process can influence the nature and composition of the mixture of products and byproducts formed. As well known in the art, iron-based F-T catalysts have high water gas shift activity and tend to favor reaction (3). In contrast, cobalt-based F-T catalysts tend to favor reaction (1), and thus enable significant amounts of water to be generated due to their relatively low water gas shift activity.

The steam generated during F-T reactions with high-temperature and high-pressure employing cobalt-based catalysts, re-oxidizes a portion of small-sized cobalt particles, or the catalyst particles are disintegrated into submicron-sized particles in a slurry reactor, thus reducing the long-term stability of the catalysts. Generation of submicron-sized catalyst particles, which takes place mainly in the slurry reactor, has the following undesirable repercussions:
1) complete removal of submicron-sized catalyst particles from the product tends to be difficult;
2) the lifetime of the catalyst can be reduced due to attrition of the catalyst in the slurry reactor; and
3) regeneration of recovered cobalt catalyst tends to be hindered, and the loss of costly cobalt metal can arise.

Meanwhile, iron-based F-T catalysts have advantages in that iron is considerably less expensive than cobalt and that the iron-based catalysts have inherently high activity. Also, because of their high water-gas shift activity, they allow F-T reactions to easily occur even at a low molar ratio of syngas (H₂/CO = 0.5-0.7) produced from coal. Thus, the high water-gas shift activity enables iron-based F-T catalysts to process low H₂/CO ratio syngas.

On the other hand, cobalt-based catalysts perform F-T reactors using natural gas-derived syngas having a high hydrogen/carbon monoxide molar ratio (H₂/CO = 1.6-2.2). When iron-based catalysts are used in F-T reactions, C₂-C₄ olefins are more frequently produced than liquid and waxy paraffinic hydrocarbons. Such C₂-C₄ olefins can be used as raw materials for a variety of chemical or petrochemical products.

Iron-based catalysts for F-T synthesis can be prepared by fusion or precipitation. The iron-based catalysts precipitated in an aqueous solution consist of iron hydroxides and iron oxides and are prepared through the precipitation, washing, drying and calcining processes. The iron-based catalysts prepared by precipitation as described above can provide liquid products of high viscosity in slurry-phase reactors. However, there is a disadvantage in that the activity of the catalysts is reduced, because the content of catalyst supports is increased in order to realize catalyst attrition resistance.

Meanwhile, iron catalysts prepared by fusion of iron ore are prepared by adding promoters to the melted oxide. Such iron catalysts have excellent attrition resistance, but show activity lower than that of the iron catalysts prepared by precipitation. Indeed, the activity of the fused iron catalysts has been measured in slurry-phase reactors as half that of precipitated iron catalysts (Fuel Processing Technology, Vol. 30, pp. 83, (1992)).

Iron-based catalysts for F-T reactions can also be prepared by a spray-drying method, and in this case, the catalyst attrition resistance is improved. It was reported that the use of the spray-drying method improved the physical strength of iron F-T catalysts without compromising the catalyst activity (Industrial & Engineering Chemistry Research, vol. 40, pp. 1065, (2001)).

Iron-based catalysts may contain at least one promoter element to assist in CO adsorption and/or iron reduction. With respect to precipitated iron catalysts, potassium is added as a promoter to increase catalytic activity and yield products with higher molecular weight. The effects of potassium on the behavior of iron catalysts may be summarized as follows:
1) potassium results in an increase in the average molecular weight of hydrocarbon products, leading to a higher α value;
2) potassium increases the olefin/paraffin ratio in the hydrocarbon products;
3) potassium suppresses the deactivation of the catalyst;
   and
4) the optimum concentration of potassium increases F-T activity and decreases the selectivity to methane.

In addition to potassium, a copper promoter is generally introduced into iron-based F-T catalysts to facilitate the reduction of the iron. Copper tends to be more effective than potassium in increasing the rate of the F-T reaction but tends to attenuate water-gas shift activity, thus not being able to maintain the H₂/CO ratio suitable for F-T reaction. In order to overcome this shortcoming, unsupported single phase iron manganese spinels which are doubly promoted with both copper and a Group IA or IIA metal may be used to selectively synthesize C₅₊ hydrocarbons at a high conversion of carbon monoxide (U.S. Patent No. 5118715).

A binder together with a promoter may be added as a structural stabilizer to an iron catalyst to provide a large surface area for the dispersion and stabilization of small metal particles. For example, silica is added as a binder to precipitated iron catalysts, especially for those used in fixed-bed reactors; however, its usefulness in slurry-phase reactors has not yet been proven. The presence of silica in iron-based catalysts for F-T synthesis may influence the characteristics of the catalyst as follows:
1) the concentration of iron in the catalyst is reduced causing greater amounts of catalytic solid to be used which in turn hinders mass transfer;
2) the concentration of active metal sites is increased as a result of the maintenance of high metal dispersions; and
3) aging characteristics of the catalyst are improved.

It was reported that coprecipitation with cobalt in the preparation of iron catalysts for F-T synthesis in slurry-phase reactors resulted in an increase in the selectivity to light olefins (U.S. Patent No. 4624967; and Applied Catalysis A: General, vol. 296, pp. 222 (2005)). It was reported that it is preferable to add potassium to this iron catalyst in order to increase the reactivity of the catalyst and the selectivity to light olefins.

As catalyst systems which are used in F-T synthesis for the selective production of light olefins from syngas, catalysts which contain a zeolite having a specific surface area of 200-500 m²/g and a Si/Al molar ratio of less than 50 may be used after conversion of the zeolite into a proton-type zeolite or after ion-exchange or impregnation of the zeolite with single or dual metal precursors of IA, IIA, Zr, P and lanthanide.

It is known that Fe-Cu-(Al)-K catalysts are used which contain, based on the weight of zeolite: 10-70 wt% of Fe (showing activity in the hydrogenation of carbon monoxide), 1-10 wt% of Cu, 0-20 wt% of Al and 1-10 wt% of K.

The present inventors have conducted studies to develop a method for preparing a catalyst which has increased activity and stability in F-T synthesis compared to Fe-Cu-Al-K catalysts reported in the prior art, which shows a high selectivity for light olefins, which utilizes a calcium oxide prepared by a sol-gel process to promote the dispersion and reduction of iron, and which shows decreased deactivation in F-T reaction conditions, leading to increased catalyst stability.

### [Disclosure]

### [Technical Problem]

The present invention has been made in order to provide a method for preparing a catalyst system suitable for the selective production of light olefins from syngas prepared from natural gas, wherein an iron-based catalyst is incorporated by impregnation into calcium oxide prepared by a sol-gel method in order to increase the yield of C₂-C₄ olefins.

The present invention relates to a method for preparing F-T catalysts for the direct production of light olefins from syngas , which utilize as a support a calcium oxide prepared by a sol-gel process. The method of the present invention provides a catalyst which increases the conversion of carbon monoxide and the yield of light olefins and shows improved stability.

### [Technical Solution]

In the frame of the present invention an iron-based F-T catalyst is disclosed which is comprising: 100 parts by weight of a calcium oxide support; 10-70 parts by weight of Fe; and 1-10 parts by weight of one metal selected from the group consisting of Cu, Co and Mn.

Further, in the frame of the present invention a method for preparing a calcium oxide support for iron-oxide F-T catalysts is disclosed, the method comprising the steps of: 1) dissolving citric acid in ethylene glycol; 2) adding an aqueous solution of a calcium precursor to the citric acid/ethylene glycol solution, stirring the mixed solution, and heating the stirred solution while completely removing water therefrom; and 3) performing a stepwise calcination of the material of step 2) from which the water has been removed.

More specifically, in the course of the present invention a method for preparing the calcium oxide support for iron-oxide F-T. catalysts is disclosed, wherein the calcium precursor is calcium nitrate (Ca(NO₃)₂·4H₂O).

More specifically, in the course of the present invention a method for preparing the calcium oxide support for iron-oxide F-T catalysts is disclosed, wherein the heating in step 2) is performed at a temperature ranging from 100°C to 150°C.

More specifically, in the course of the present invention a method for preparing the calcium oxide support for iron-oxide F-T catalysts is disclosed, wherein the calcium oxide support has a specific surface area of 5 to 50 m²/g. The present invention relates to a method for preparing an iron-oxide F-T catalyst, the method comprising the steps of: 1) dissolving citric acid in ethylene glycol; 2) adding an aqueous solution of a calcium precursor to the citric acid/ethylene glycol solution, stirring the mixed solution, and heating the stirred solution while completely removing water therefrom; 3) performing a stepwise calcination of the material of step 2) from which the water has been removed; and 4) incorporating at least three metal precursors including an iron precursor and a potassium precursor into the calcium oxide support by impregnation or coprecipitation, wherein the calcium oxide support has a specific surface area of 5 to 50 m²/g and the F-T catalyst comprising 100 parts by weight of a calcium oxide support; 10-70 parts by weight of Fe; 1-10 parts by weight of K; and 1-10 parts by weight of at least one metal element selected from the group consisting of Cu, Co and Mn.

More specifically, the present invention relates to the method for preparing the iron-based F-T catalyst, wherein the calcium precursor is calcium nitrate (Ca(NO₃)₂·4H₂O).

More specifically, the present invention relates to the method for preparing the iron-based F-T catalyst, wherein the iron precursor is one or a mixture of two or more selected from the group consisting of Fe (II) and Fe (III) precursors, including iron nitrate hydrate (Fe(NO₃)₃·9H₂O), iron acetate (Fe **(**CO₂CH₃) ₂), iron oxalate hydrate (Fe(C₂O₄)₃·₆H₂O), iron acetylacetonate (Fe (C₅H₇O₂)₃) and iron chloride (FeCl₃).

More specifically, the present invention relates to the method for preparing the iron-based F-T catalyst, wherein the metal precursors additionally include a metal salt of at least one selected from the group consisting of copper, cobalt and manganese.

More specifically, the present invention relates to the method for preparing the iron-based F-T catalyst, which further comprises a step of calcining Thus prepared catalyst at a temperature ranging from 300 °C to 700°C.

### [Advantageous Effects]

In the development of the STO process and catalyst technologies for selectively producing light olefins, which are basic raw materials in the petrochemical industry, while coping with skyrocketing oil prices, the development of F-T catalysts efficient for the STO reactions can become an important factor in ensuring competitiveness.

Particularly, as a result of the improvement of the method for preparing iron-based F-T catalysts, the thermal efficiency and carbon utilization efficiency of the entire process can be improved.

In addition, it is considered that the inventive method for preparing iron-based F-T catalyst showing stable product stability and low deactivation in the production of light olefins will greatly contribute to the development of cost-effective STO processes in the future.

### [Description of Drawings]

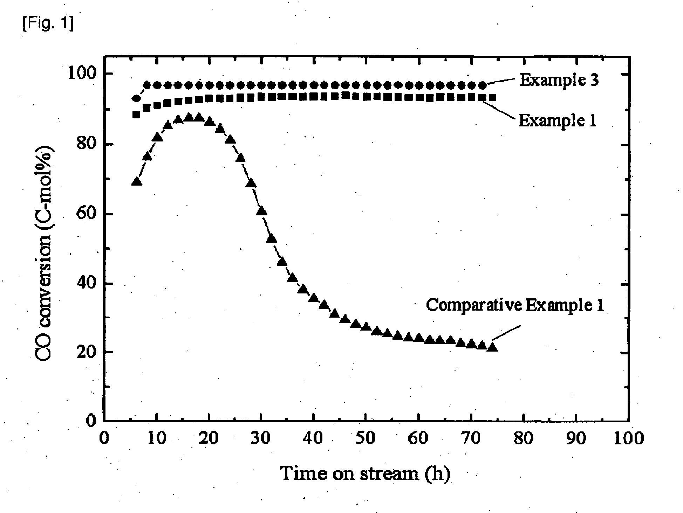

FIG. 1 shows carbon monoxide conversion as a function of time in reactions employing F-T catalysts prepared in Examples 1 and 2 and Comparative Example 1.

### [Best Mode]

The present invention has been made in order to provide a method for preparing a catalyst system suitable for the selective production of light olefins from syngas prepared from natural gas, wherein an iron-based catalyst is incorporated by impregnation into a calcium oxide prepared by a sol-gel process in order to increase the yield of C₂-C₉ olefins.

Hereinafter, the present invention will be described in detail.

In the frame of the present invention an iron-based F-T catalyst is disclosed comprising: 100 parts by weight of a calcium oxide support; 10-70 parts by weight of Fe; 1-10 parts by weight of K; and 1-10 parts by weight of one metal element selected from the group consisting of Cu, Co and Mn.

As generally known in the art, the use of iron-based catalysts in F-T reactions for the production of liquid hydrocarbons from syngas shows wide product distribution and low olefin selectivity because of the F-T synthesis mechanism. Specifically, it is known that the selectivity for C₂-C₄ hydrocarbons is about 30% and the selectivity for olefins is about 80%. Also, it is known that the maximum yield of C₂-C₄ hydrocarbons in F-T reactions cannot exceed 56% as calculated according to an Anderson-Schulz-Flory polymerization model due to the characteristics of CH₂ chain growth mechanism.

Korean Patent Application No. 10-2007-0103677 previously filed by the applicant discloses processes for producing light olefins from syngas, wherein high-boiling-point hydrocarbons are secondarily pyrolyzed or catalytically cracked using a zeolite-based acid catalyst, and the selectivity to olefins is improved through dehydrogenation. The invention disclosed in said Korean patent application suggests a process capable of increasing the production of light olefin while maximizing the utilization of byproducts by recycling reaction byproducts (methane and carbon dioxide) to a reforming process in order to improve the yield of C₂-C₄ hydrocarbons and increase carbon utilization efficiency on a catalyst prepared by impregnating or coprecipitating an iron catalyst into zeolite.

Iron-based catalysts which are used in the STO process can be contained in a support such as silica, alumina or zeolite by impregnation or coprecipitation. When zeolite is used as the support, zeolite having a specific surface area of 200-500 m²/g and a Si/Al molar ratio of less than 50 may be used after conversion to proton-type zeolite or after ion exchange or impregnation with single or double metal precursors of IA, IIA, Zr, P and lanthanide.

A Fe-Cu-Al-K catalyst containing, based on the weight of a support, 10-50 wt% of Fe, as a component showing activity in the hydrogenation of carbon monoxide, 1-10 wt% of Cu, 0-20 wt% of Al and 1-5 wt% of K, is used in F-T reactions to produce hydrocarbons.

The iron-based catalyst system disclosed in the present invention contains, as an active ingredient, a Fe-Cu (or Co and Mn)-K catalyst containing, based on the weight of calcium oxide, 10-70 wt% of Fe, as a component showing activity in the hydrogenation of carbon monoxide, 1-10 wt% of Cu (or Co and Mn) and 1-10 wt% of K.

The iron-based catalyst disclosed in the frame of the present invention and which is used in the F-T process can be contained in a calcium oxide support by impregnation or coprecipitation before use.

Examples of the iron precursor which can be used in the frame of the present invention include Fe (II) and Fe (III) precursors, such as iron nitrate hydrate (Fe(NO₃)₃·H₂O), iron acetate (Fe(CO₂CH₃)₂), iron oxalate hydrate (Fe(C₂O₄)₃·H₂O), iron acetylacetonate (Fe(C₅H₇O₂)₃) and iron chloride (FeCl₃).

Also, a copper, cobalt or manganese precursor which is added to improve the reduction of iron may be selected from among acetate-, nitrate- and chloride-based precursors, and examples of a potassium precursor which is used to increase the selectivity to olefins include K₂CO₃ and KOH. An Al component may be additionally used to improve the dispersion of Fe-Cu (or Co and Mn) components, and in this case, the Al precursor may be selected from aluminum acetate- and aluminum nitrate-based precursors. The Fe-Cu (or Co and Mn)-K catalyst prepared using at least three metal precursors including Fe and Cu is loaded onto a zeolite support by impregnation or coprecipitation before use, and thus prepared catalyst needs to be calcined at a temperature ranging from 300°C to 700°C to stabilize the structure thereof in advance.

In the frame of the present invention a method for preparing a calcium oxide support for iron-oxide F-T catalysts is disclosed, the method comprising the steps of: 1) dissolving citric acid in ethylene glycol; 2) adding an aqueous solution of a calcium precursor to the citric acid/ethylene glycol solution, stirring the mixed solution, and heating the stirred solution while completely removing water therefrom; and 3) performing a stepwise calcination of the material of step 2) from which the water has been removed.

More specifically, in the method for preparing a calcium oxide support for iron-oxide F-T catalysts, the calcium precursor is calcium nitrate (Ca(NO₃)_{2·}4H₂O).

More specifically, in the method for preparing a calcium oxide support for iron-oxide F-T catalysts, the heating in step 2) is performed at a temperature ranging from 100°C to 150 °C.

In the frame of the present invention, the iron-based catalyst which is used in the F-T process can be contained in a calcium oxide support by impregnation or coprecipitation. For the preparation of the calcium oxide support, a calcium precursor is introduced. The method for preparing the calcium oxide support will now be described in detail.

Calcium oxide for use as the support was prepared by a sol-gel process in the following manner. First, 312.82 g of citric acid was dissolved by stirring in 263.49 g of ethylene glycol at 60°C for 30 minutes.

As the calcium precursor, calcium nitrate was used, but the scope of the present invention is not limited thereto. 30.06 g of calcium nitrate (Ca(NO₃)_{2·}H₂O) was completely dissolved in less than 30 ml of water and was then added slowly to Thus prepared citric acid/ethylene glycol solution. The mixed solution was stirred at 60°C for 30 minutes, and then heated to completely remove water from the solution.

The heating of the stirred solution is preferably performed at a temperature ranging from 100°C to 150°C. If the heating temperature is lower than 100°C it will be difficult to remove water, because the temperature is lower than the boiling point of water, and if the heating temperature is higher than 150°C, calcium oxide particles will not be uniformly synthesized due to the rapid evaporation of water at high temperature.

Thus prepared sol-state material was calcined stepwise with a heating rate of 5 °C /min and maintained at each temperatures of 100, 150, 200 and 300°C for 1 hour. To maximize the surface area of the support, the sol-state material was maintained at 400°C for 2 hours. Finally, the material was calcined at 500°C for 4 hours. If thus prepared calcium oxide sol is calcined while rapidly increasing the calcination temperature, a wide specific surface area cannot be obtained with the aggregation of the calcium oxide precursor. In contrast, if the calcination of the sol is performed while sequentially increasing the temperature, a wide specific surface area can be obtained.

Specifically, the present invention discloses a method for preparing the calcium oxide support for F-T catalysts, wherein the calcium oxide support has a specific surface area of 5-50 m²/g_{.}

According to the preparation method of the present invention, the specific surface area of the calcium oxide support having a specific surface area of 5-50 m²/g is obtained. The larger the specific surface area of the calcium oxide support, the better the dispersion of the active ingredient iron. If the specific surface area of the calcium oxide support is smaller than 5 m²/g, the dispersion of the iron will be decreased. Meanwhile, if the specific surface area of the calcium oxide support exceeds 50 m²/g, the strength of the catalyst will be reduced.

The present invention relates to a method for preparing an iron-based F-T catalyst, the method comprising the steps of: 1) dissolving citric acid in ethylene glycol; 2) adding an aqueous solution of a calcium precursor to the citric acid/ethylene glycol solution, stirring the mixed solution, and heating the stirred solution while completely removing water therefrom; 3) performing a stepwise calcination of the material of step 2) from which the water has been removed; and 4) incorporating at least three metal precursors including an iron precursor and a potassium precursor into the calcium oxide support by impregnation or coprecipitation, wherein the calcium oxide support has a specific surface area of 5-50 m²/g, and the F-T catalyst comprising 100 parts by weight of a calcium oxide support; 10-70 parts by weight of Fe; 1-10 parts by weight of K; and 1-10 parts by weight of at least one metal element selected from the group consisting of Cu, Co and Mn.

More specifically, the present invention relates to the method for preparing the iron-based F-T catalyst, wherein the calcium precursor is calcium nitrate (Ca(NO₃)₂·4H₂O).

After dissolving ethylene glycol in citric acid, the aqueous solution of the calcium precursor is added to the citric acid/ethylene glycol solution. The mixed solution is stirred and heated while completely removing water from the solution. For the preparation of the calcium oxide precursor, the calcium precursor is introduced. As the calcium nitrate, calcium nitrate is preferably used, but the scope of the present invention is not limited thereto. The compound from which the water has been completely removed is calcined stepwise, thus obtaining a calcium oxide support.

The method of preparing the F-T catalyst by incorporating the iron-based active ingredient into the prepared calcium oxide can be performed in the following two manners.

First, into the calcium oxide support prepared by the sol-gel process and calcined, the iron precursor, the copper precursor and the potassium precursor are incorporated by simultaneous impregnation or sequential impregnation, thereby preparing a Fe-Cu (or Co and Mn)-K catalyst containing, based on the weight of the calcium oxide, 10-70 wt% of Fe, 1-10 wt% of Cu (or Co and Mn) and 1-10 wt% of K.

In another method for preparing the STO catalyst, in order to incorporate Fe-Cu (or Co and Mn)-K components into the slurry of the calcium oxide prepared by the sol-gel process, at least three metal precursors including an iron precursor and a potassium precursor is used to prepare a mixed solution of metal precursors. Thus prepared metal precursor solution is coprecipitated with the calcium oxide in a solution, such that the final pH reaches the range from 7 to 8. Then, the solution is aged at a temperature ranging from 40°C to 90°C, and the precipitate is filtered and washed, thereby preparing the above-described catalyst. In order to maintain a pH of 7-8 in the coprecipitation, a basic precipitant may be used. Preferred examples of the basic precipitant which can be used in the present invention include sodium carbonate, calcium carbonate, ammonium carbonate and ammonia water.

The aging time of the catalyst is 0.1-10 hours, and preferably 0.5-8 hours, and this aging time range assists in the formation of an iron-based catalyst having high activity. If the aging time is shorter than 0.5, the dispersion of the Fe-Cu (or Co and Mn)-Al components will be decreased, thus causing a disadvantage in terms of the F-T reaction, and if the aging time exceeds 10 hours, the size of the Fe-Cu (or Co and Mn)-Al particles will be decreased, and the synthesis time will be increased, leading to cost-ineffectiveness.

The hybrid catalyst of the Fe-Cu (or Co and Mn)-Al catalyst with the calcium oxide is loaded with 1-10 wt% of potassium, thereby preparing a desired STO catalyst.

According to the present invention, in the method for preparing the iron-based F-T catalyst, the calcium oxide support has a specific surface area of 5-50 m²/g.

According to the preparation method of the present invention, the calcium oxide support having a specific surface area of 5-50 m²/g is obtained, and the larger the specific surface area, the better the dispersion of iron. If the specific surface area of the calcium oxide support is smaller than 5 m²/g, the dispersion of iron will be reduced, and if the specific surface area of the calcium oxide support exceeds 50 m²/g, the strength of the catalyst will be reduced.

More specifically, in the method of the present invention for preparing the iron-based F-T catalyst, the iron precursor is one or a mixture of two or more selected from the group consisting of Fe (II) and Fe (III) precursors, such as iron nitrate hydrate (Fe(NO₃)₃·9H₂O), iron acetate (Fe(CO₂CH₃)₂), iron oxalate hydrate (Fe(C₂O₄)₃·6H₂O), iron acetylacetonate (Fe (C₅H₇O₂) ₃) and iron chloride (FeCl₃).

Also, the present invention relates to the preparation method wherein the metal precursors include a metal salt of one selected from the group consisting of copper, cobalt and manganese.

In the preparation method of the present invention, a mixed solution of metal precursors is prepared using an iron precursor which is one or a mixture of two or more selected from among Fe (II) and Fe (III) precursors, such as iron nitrate hydrate (Fe(NO₃)₃·9H₂O), iron acetate (Fe(CO₂CH₃)2), iron oxalate hydrate (Fe(C₂O₄)₃·6H₂O), iron acetylacetonate (Fe(C₅H₇O₂)₃) and iron chloride (FeCl₃) Fe (II) and Fe (III) precursors, and copper and aluminum precursors selected from among acetate-, nitrate- and chloride-based precursors, which are added to improve the reduction of iron.

More specifically, the present invention relates to the method for preparing the iron-based F-T catalyst, which further comprises a step of calcining thus prepared catalyst at a temperature ranging from 300 to 700°C.

The catalyst prepared according to the above method may be dried in an oven at more than 100°C for about one day, and then calcined at a temperature between 300°C and 700°C, and preferably 400°C and 600°C, in order to use the catalyst for the STO reactions. If the calcination temperature is lower than 300°C, the Fe-Cu-Al-K components cannot be converted to oxide forms, resulting in decrease in the dispersion of the active ingredient. Meanwhile, if the calcination temperature is higher than 700°C, active sites can be reduced due to the aggregation of the Fe-Cu-Al-K components. For this reason, the calcination temperature needs to be maintained in the above temperature range.

The STO catalyst is used in catalytic reactions, after it is reduced in a fixed-bed, fluidized-bed or slurry-phase reactor at a temperature ranging from 200 to 700°C in a hydrogen atmosphere. The reduced STO catalyst is used in conditions similar to those of general STO reactions. Specifically, the STO catalyst is preferably used at a temperature between 250°C and 400°C at a pressure of 5-60 kg/cm² at a space velocity of 500-10000 h⁻¹, but the scope of the present invention is not limited thereto.

### [Mode for Invention]

Hereinafter, the present invention will be described in further detail with reference to examples in connection with STO reactions, but the scope of the present invention is not limited only to these examples.

### Examples

### Example 1. Preparation of 4 wt% K / 20 wt% Fe - 2 wt% Co / CaOx catalyst. (stepwise impregnation)

### Preparation of calcium oxide

First, calcium oxide as a support was prepared by a sol-gel process. For this purpose, 312.82 g of citric acid was dissolved by stirring in 263.49. g of ethylene glycol at 60°C for 30 minutes. 30.06 g of calcium nitrate hydrate (Ca(NO₃)₂·H₂O) as a calcium precursor was completely dissolved in the minimum amount (less than 30 ml) of water and added slowly to Thus prepared citric acid/ethylene glycol solution. Herein, the citric acid was used in an amount 10 times the number of moles of calcium, and the ethylene glycol in an amount 40 times the moles of calcium. The mixture was stirred at 60°C for 30 minutes, and then stirred at 130 °C for 5 hours to completely remove water from the stirred solution. Thus obtained sol-state material was maintained for 1 hour at temperatures of 100, 150, 200 and 300°C and increased its temperature with a heating rate of 5°C /min between each temperature. Then, the material was additionally maintained at 400°C for 2 hours in order to maximize the surface area of the support, and finally maintained at 500°C for 4 hours, thus calcining the sol-state material.

### Preparation of catalyst

3 g of Thus prepared powdery calcium oxide support, 4.406 g of iron nitrate hydrate (Fe (NO₃)_{3·}H₂O) as an iron precursor and 0.305 g of cobalt nitrate hydrate (Co (NO₃)₂·H₂O) as a cobalt precursor were dissolved in 60 ml of distilled water. The solution was stirred at 60°C for at least 6 hours and distilled under reduced pressure to remove water in a rotary evaporator. Thus prepared iron-cobalt/calcium oxide catalyst was added to 60 ml of an aqueous solution containing 0.213 g of a potassium precursor (K₂CO₃) dissolved therein, and was then stirred at 60°C for at least 6 hours. The stirred solution was dried at 105°C for at least 12 hours, and then calcined at 500°C for 5 hours in an air atmosphere, thereby preparing a potassium/iron-cobalt/calcium oxide catalyst. Thus prepared catalyst had a composition comprising 4 wt% of K / 20 wt% of Fe - 2 wt% of Co / CaOx based on metal content and a specific surface area of 4.2 m²/g

A 1.27 cm (1/2-inch) stainless fixed-bed reactor was charged with 0.3 g of the catalyst before the start of a reaction. The catalyst was reduced at 450°C for 12 hours in a hydrogen atmosphere (5 vol% H₂/He), and then the reactants, carbon monoxide: hydrogen: argon (internal standard), were set at a ratio of 31. 7: 63.3: 5 under conditions of a temperature of 300°C, a pressure of 10 kg/cm² and a space velocity of 2000 L/kg_{cat}hr and introduced into the reactor. Then, the reactants were subjected to an F-T reaction.

The activity of the catalyst was stabilized and maintained after 60 hours from the start of the reaction, and the results of the reaction are shown in Table 1 below as the average of values measured during 10 hours after the 60-hour reaction time.

### Example 2: Preparation of 4 wt% K / 20 wt% Fe - 4 wt% Co / CaOx catalyst (stepwise impregnation)

A catalyst was prepared in the same manner as in Example 1, except that cobalt nitrate hydrate (Co(NO₃)₂·H₂O) among the metal precursors was used in an amount of 0.610 g. Thus prepared catalyst had a composition comprising 4 wt% of K / 20 wt% of Fe - 4 wt% of Co / CaOx based on metal content and a specific surface area of 6.7 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Example 3: Preparation of 20 wt% Fe - 2 wt% Co - 4 wt% K / CaOx catalyst (Coimpregnation)

3 g of a powdery calcium oxide support prepared in the same manner as in Example 1, 4.406 g of iron nitrate hydrate (Fe(NO₃)₃·H₂O) as an iron precursor, 0.305 g of cobalt nitrate hydrate (Co (NO₃) ₂ ·H₂O) as a cobalt precursor and 0.213 g of (Co (NO₃)₂ ·H₂O) were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-cobalt-potassium/calcium oxide catalyst. Thus prepared catalyst had a composition comprising 20 wt% of Fe - 2 wt% of Co - 4 wt% of K/CaOx based on metal content and a specific surface area of 14.2 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Example 4: Preparation of 20 wt% Fe - 2 wt% Cu - 4 wt% K / CaOx catalyst (Coimpregnation)

3 g of a powdery calcium oxide support prepared in the same manner as in Example 1, 4.406 g of iron nitrate hydrate (Fe(NO₃)_{3·}H₂O) as an iron precursor, 0.291 g of copper nitrate hydrate (Cu(NO₃)₂·H₂O) as a copper precursor and 0.213 g of potassium carbonate (K₂CO₃) as a potassium precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-copper-potassium/calcium oxide catalyst. Thus prepared catalyst had a composition comprising 20 wt% of Fe - 2 wt% of Cu- 4 wt% of K / CaOx based on metal content and a specific surface area of 14.0 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Example 5: Preparation of 20 wit% Fe - 2 wt% Mn - 4 wt% K / CaOx catalyst (Coimpregnation)

3 g of a powdery calcium oxide support prepared in the same manner as in Example 1, 4.406 g of iron nitrate hydrate (Fe(NO₃)_{3·}H₂O) as an iron precursor, 0.320 g of manganese nitrate hydrate (Mn(NO₃)₂·H₂O) as a manganese precursor and 0.213 g of potassium carbonate (K₂CO₃) as a potassium precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-manganese-potassium/calcium oxide catalyst. Thus prepared catalyst had a composition comprising 20 wt% of Fe - 2 wt% of Mn - 4 wt% of K / CaOx based on metal content and a specific surface area of 24.1 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Example 6: Preparation of 30 wt% Fe - 3 wt% Co - 6 wt% K / CaOx catalyst (Coimpregnation)

3 g of a powdery calcium oxide support prepared in the same manner as in Example 1, 6.609 g of iron nitrate hydrate (Fe (NO₃)₃ ·H₂O) as an iron precursor, 0.457 g of cobalt nitrate hydrate (Co (NO₃)_{2·}H₂O) as a cobalt precursor and 0.319 g of potassium carbonate (K₂CO₃) as a potassium precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-cobalt-potassium/calcium oxide catalyst. Thus prepared catalyst had a composition comprising 30 wt% of Fe - 3 wt% of Co - 6 wt% of K / CaOx based on metal content and a specific surface area of 12.3 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Example 7: Preparation of 50 wt% Fe - 5 wt% Co - 10 wt% K / CaOx catalyst (Coimpregnation)

3 g of a powdery calcium oxide support prepared in the same manner as in Example 1, 11.015 g of iron nitrate hydrate (Fe(NO₃)₃·H₂O) as an iron precursor, 0.763 g of cobalt nitrate hydrate (Co(NO₃)₂·H₂O) as a cobalt precursor and 0.532 g of potassium carbonate (K₂CO₃) as a potassium precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-cobalt-potassium/calcium oxide catalyst. Thus prepared catalyst had a composition comprising 50 wt% of Fe - 5 wt% of Co - 10 wt% of K / CaOx based on metal content and a specific surface area of 11.6 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Example 8: Preparation of 70 wt% Fe - 7 wt% Co - 14 wt% K / CaOx catalyst Coimpregnation)

3 g of a powdery calcium oxide support prepared in the same manner as in Example 1, 15.421 g of iron nitrate hydrate (Fe(NO₃)₃·H₂O) as an iron precursor, 1.067 g of cobalt nitrate hydrate (Co(NO₃)2·H₂O) as a cobalt precursor and 0.745 g of potassium carbonate (K₂CO₃) as a potassium precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-cobalt-potassium/calcium oxide catalyst. Thus prepared catalyst had a composition comprising 70 wt% of Fe - 7 wt% of Co - 14 wt% of K / CaOx based on metal content and a specific surface area of 11.8 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Examples

### Comparative Example 1: Preparation of 20 wt% Fe - 2 wt% Cu - 4 wt% K / α-Al₂O₃ catalyst (Coimpregnation)

3 g of an alpha-alumina support in place of the powdery calcium oxide prepared in Example 1, 4.406 g of iron nitrate hydrate (Fe(NO₃)₃·H₂O) as an iron precursor, 0.291 g of copper nitrate hydrate (Cu (NO₃)₂ ·H₂O) as a cobalt precursor and 0.213 g of potassium carbonate (K₂CO₃) as a potassium precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-copper-potassium/calcium oxide catalyst. Thus prepared catalyst had a composition comprising 20 wt% of Fe - 2 wt% of Cu - 4 wt% of K / α-Al₂O₃ based on metal content and a specific surface area of 4.2 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Example 2: Preparation of 20 wt% Fe / CaOx catalyst (Impregnation)

3 g of a powdery calcium oxide support prepared in the same manner as in Example 1, and 4.406 g of iron nitrate hydrate (Fe(NO₃)₃·H₂O) as an iron precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron/calcium oxide catalyst. Thus prepared catalyst had a composition comprising 20 wt% of Fe / CaOx based on metal content and a specific surface area of 6.8 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Example 3: Preparation of 20 wt% Fe - 2 wt% Co / CaOx catalyst (Coimpregnation)

3 g of a powdery calcium oxide support prepared in the same manner as in Example 1, 4.406 g of iron nitrate hydrate (Fe(NO₃)₃·H₂O) as an iron precursor and 0.305 g of cobalt nitrate hydrate (Co(NO₃)₂·H₂O) as a cobalt precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-cobalt-potassium/calcium oxide catalyst. Thus prepared catalyst had a composition comprising 20 wt% of Fe - 2 wt% of Co / CaOx based on metal content and a specific surface area of 4.0 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Example 4: Preparation of 4 wt% K / 20 wt% Fe / CaOx catalyst (stepwise impregnation)

3 g of a powdery calcium oxide support prepared in the same manner as in Example 1 and 4.406 g of iron nitrate hydrate (Fe(NO₃)₃·H₂O) as an iron precursor were dissolved in 60 ml of distilled water. The solution was stirred at 60°C for at least 6 hours and distilled under reduced pressure to remove water.

Thus prepared iron/calcium oxide catalyst was added to 60 ml of an aqueous solution containing 0.213 g of a potassium precursor (K₂CO₃) dissolved therein, and was then stirred at 60°C for at least 6 hours. The stirred solution was dried at 105°C for at least 12 hours, and then calcined at 500°C for 5 hours in an air atmosphere, thereby preparing a potassium-iron /calcium oxide catalyst. Thus prepared catalyst had a composition comprising 4 wt% of K / 20 wt% of Fe / CaOx based on metal content and a specific surface area of 4.8 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Example 5: Preparation of 20 wt% Fe - 2 wt% Cu - 4 wt% K / SiO₂ catalyst (Coimpregnation)

3 g of Davisil silica (specific surface area: 300 m²/g; and particle size: 150-250 microns CO-100 mesh)) in place of the powdery calcium oxide prepared in Example 1, 4.406 g of iron nitrate hydrate (Fe(NO₃)₃·H₂O) as an iron precursor, 0.291 g of copper nitrate hydrate (Cu(NO₃)₂·H₂O) as a cobalt precursor and 0.213 g of potassium carbonate (K₂CO₃) as a potassium precursor were dissolved in 60 ml of distilled water. The solution was stirred at 60°C for at least 6 hours and distilled under reduced pressure to remove water. Then, the resulting material was dried at 105°C for at least 12 hours, and then calcined at 500°C for 5 hours in an air atmosphere, thereby preparing a iron-copper-potassium/calcium oxide catalyst. Thus prepared catalyst had a composition comprising 20 wt% of Fe - 2 wt% of Cu - 4 wt% of K / SiO₂ based on metal content and a specific surface area of 5.0 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Example 6: Preparation of 20 wt% Fe - 2 wt% Cu - 4 wt% K / CaOx catalyst (Coimpregnation)

3 g of reagent-grade calcium oxide (Duksan; 98%) in place of the powdery calcium oxide prepared in Example 1, 4.406 g of iron nitrate hydrate (Fe (NO₃)3 ·H₂O) as an iron precursor, 0.291 g of copper nitrate hydrate (Cu(NO₃)₂ ·H₂O) as a cobalt precursor and 0.213 g of potassium carbonate (K₂CO₃) as a potassium precursor were dissolved in 60 ml of distilled water. The solution was stirred at 60°C for at least 6 hours and distilled under reduced pressure to remove water. Then, the resulting material was dried at 105°C for at least 12 hours, and then calcined at 500°C for 5 hours in an air atmosphere, thereby preparing an iron-copper-potassium/calcium oxide catalyst. Thus prepared catalyst had a composition comprising 20 wt% of Fe - 2 wt% of Cu - 4 wt% of K/CaOx based on metal content and a specific surface area of 14.6 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Example 7: Preparation of 20 wt% Fe - 2 wt% Co - 4 wit% K / CaOx catalyst (Coimpregnation)

3 g of reagent-grade calcium oxide (Duksan; 98%) in place of the powdery calcium oxide prepared in Example 1, 4.406 g of iron nitrate hydrate (Fe(NO₃)₃ ·H20) as an iron precursor, and 0.305 g of cobalt nitrate hydrate (Co(NO₃)₂·H₂O) as a cobalt precursor and 0.213 g of potassium carbonate (K₂CO₃) as a potassium precursor were dissolved in 60 ml of distilled water. The solution was stirred at 60°C for at least 6 hours and distilled under reduced pressure to remove water in a rotary evaporator. Then, the resulting material was dried at 105°C for at least 12 hours, and then calcined at 500°C for 5 hours in an air atmosphere, thereby preparing an iron-cobalt-potassium/calcium oxide catalyst. Thus prepared catalyst had a composition comprising 20 wt% of Fe - 2 wt% of Co - 4 wt% of K / CaOx based on metal content and a specific surface area of 14.8 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Example 8: Preparation of 4 wt% K / 20 wt% Fe - 2 wt% Co - CaOx catalyst (Coprecipitation)

First, an iron-cobalt-calcium oxide catalyst was prepared by coprecipitation. For this purpose, 13.353 g of iron nitrate hydrate (Fe(NO₃)₃·H₂O) as an iron precursor, 0.885 g of cobalt nitrate hydrate (Co (NO₃)₂·H₂O) as a cobalt precursor and 0.523 g of calcium nitrate hydrate (Ca (NO₃)₂·H₂O) as a calcium precursor were dissolved in 100 ml of distilled water, and then coprecipitated with 100 ml of an aqueous solution containing 11.109 g of ammonia water dissolved therein at 70°C at a pH of about 7.

Thus prepared iron-cobalt-calcium oxide catalyst was added to 60 ml of an aqueous solution containing 0.646 g of a potassium precursor (K₂CO₃), and was then stirred at 60°C for at least 6 hours. The stirred solution was dried at 105 °C for at least 12 hours, and then calcined at 500°C for 5 hours in an air atmosphere, thereby preparing a potassium/iron-cobalt-calcium oxide catalyst. Thus prepared catalyst had a composition comprising 4 wt% of K / 20 wt% of Fe - 2 wt% of Co - CaOx based on metal content and a specific surface area of 8.3 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

### Comparative Example 9: Preparation of 4 wt% K / 20 wt% Fe - 2 wt% Cu - CaOx catalyst (Coprecipitation)

First, an iron-cobalt-calcium oxide catalyst was prepared by coprecipitation. For this purpose, 13.353 g of iron nitrate hydrate (Fe(NO₃)₃·H₂O) as an iron precursor, 0.842 g of cobalt nitrate hydrate (Co(NO₃)₂·H₂O) as a cobalt precursor and 0.523 g of calcium nitrate hydrate (Ca(NO₃)₂·H₂O) as a calcium precursor were dissolved in 100 ml of distilled water, and then coprecipitated with 100 ml of an aqueous solution containing 11.066 g of ammonia water dissolved therein at 70°C at a pH of about 7.

Thus prepared iron-cobalt-calcium oxide catalyst was added to 60 ml of an aqueous solution containing 0.646 g of a potassium precursor (K₂CO₃), and was then stirred at 60°C for at least 6 hours. The stirred solution was dried at 105°C for at least 12 hours, and then calcined at 500°C for 5 hours in an air atmosphere, thereby preparing a potassium/iron-cobalt-calcium oxide catalyst. Thus prepared catalyst had a composition comprising 4 wt% of K / 20 wt% of Fe - 2 wt% of Cu - CaOx based on metal content and a specific surface area of 7.5 m²/g.

An F-T reaction was performed on the catalyst in the same conditions as in Example 1. The activity of the catalyst was stabilized and maintained after 60 hours from the start of the reaction, and the results of the reaction are shown in Table 1 below as the average of values measured during 10 hours after the 60-hour reaction time.

**[Table 1]**

| | CO conversion (carbon mol%) | CO-to-CO₂ conversion (carbon mol%) | carbon selectivity to C₁/ C₂ - C₄/more than C₅ (carbon mol%) | Yield of C₂ - C₄ olefins (%) |
|---|---|---|---|---|
| Example 1 | 93.5 | 37.8 | 10.8/33.0/5.2 | 14.9 |
| Example 2 | 92.8 | 40.2 | 16.7/34.8/48.5 | 14.4 |
| Example 3 | 96.8 | 36.4 | 9.1/31.8/59.1 | 16.6 |
| Example 4 | 95.4 | 37.4 | 6.9/26.3/66.8 | 13.2 |
| Example 5 | 95.3 | 36.4 | 7.2/27.2/65.6 | 13.9 |
| Example 6 | 96.1 | 41.7 | 6.5/24.1/69.4 | 11.2 |
| Example 7 | 96.4 | 41.5 | 7.7/25.7/66.6 | 11.9 |
| Example 8 | 96.9 | 39.4 | 6.3/23.8/69.9 | 11.9 |
| Comparative Example 1 | 22.6 | 9.4 | 8.0/19.7/72.3 | 2.1 |
| Comparative Example 2 | 43.0 | 20.4 | 19.2/42.6/38.2 | 6.2 |
| Comparative Example 3 | 76.6 | 32.8 | 37.1/51.7/11.2 | 8.9 |
| Comparative Example 4 | 26.6 | 11.7 | 5.8/17.1/77.1 | 2.1 |
| Comparative Example 5 | 72.8 | 30.6 | 25.6/45.2/29.2 | 10.2 |
| Comparative Example 6 | 93.5 | 41.6 | 5.7/23.9/70.4 | 10.6 |
| Comparative Example 7 | 93.0 | 45.8 | 7.2/22.2/70.6 | 8.7 |
| Comparative Example 8 | 72.1 | 38.3 | 9.6/19.8/70.6 | 5.4 |
| Comparative Example 9 | 80.2 | 36.7 | 4.3/15.8/79.9 | 5.8 |

As can be seen in Table 1 above, the iron-based catalysts containing the calcium oxide prepared by the sol-gel process according to the present invention (Examples 1 to 8) showed a high selectivity for olefins, an increase in the yield of olefins and an increase in long-term catalyst stability compared to the iron-based catalysts containing no calcium oxide (Comparative Examples 1 and 5), the iron-based catalysts to which copper and potassium as activity promoters had not been added in the concentration range proposed in the present invention (Comparative Examples 2, 3 and 4), and the iron-based catalysts prepared using the calcium oxide prepared by processes other than the sol-gel process (Comparative Examples 6, 7, 8 and 9).

Further, as can be seen through Comparative Examples, it is advantageous that the F-T catalysts comprising the iron-based catalyst and the calcium oxide prepared by the sol-gel process additionally comprise potassium and one selected from among copper, manganese and cobalt in order to increase the reduction of the iron and reduce the selectivity to methane.

In addition, it can be seen that, in the case of the iron-based catalysts containing the calcium oxide prepared by the sol-gel method proposed in the present invention, the deactivation of the catalyst with the passage of reaction time is very low, thus ensuring long-term catalyst performance and stable process operation.

## Claims

1. A method for preparing iron-oxide F-T catalysts, the method comprising the steps of:
1) dissolving citric acid in ethylene glycol;
2) adding an aqueous solution of a calcium precursor to the citric acid/ethylene glycol solution, stirring the mixed solution, and heating the stirred solution while completely removing water therefrom;
3) performing a stepwise calcination of the material of step 2) from which the water has been removed; and
4) incorporating at least three metal precursors including an iron precursor and a potassium precursor into the calcium oxide support by coprecipitation or impregnation
wherein the calcium oxide support has a specific surface area of 5-50 m²/g, and the F-T catalyst comprising 100 parts by weight of a calcium oxide support; 10-70 parts by weight of Fe; 1-10 parts by weight of K; and 1-10 parts by weight of at least one metal element selected from the group consisting of Cu, Co and Mn.

2. The method of Claim 1, wherein the calcium precursor is calcium nitrate (Ca(NO₃)₂.4H_{2O}).

3. The method of Claim 1, wherein the iron precursor is one or a mixture of two or more selected from the group consisting of Fe (II) and Fe (III) precursors, including iron nitrate hydrate (Fe (NO₃)_{3·}9H₂O), iron acetate (Fe(CO₂CH₃)₂), iron oxalate hydrate (Fe(C₂O₄)₃.6H₂O), iron acetylacetonate (Fe(C₅H₇O₂)₃) and iron chloride.

4. The method of Claim 1, wherein the metal precursors additionally include a metal salt of one selected from the group consisting of copper, cobalt and manganese.

5. The method of Claim 1, which further comprises a step of calcining thus prepared catalyst at a temperature ranging from 300°C to 700°C.

6. The method of Claim 1, wherein the heating in step 2) is performed at a temperature ranging from 100°C to 150°C.

## Patentansprüche

1. Verfahren zur Herstellung von Eisenoxid-F-T-Katalysatoren, wobei das Verfahren die Schritte umfasst von:
1) Auflösen von Zitronensäure in Ethylenglycol;
2) Zugeben einer wässrigen Lösung einer Calcium-Vorstufe zu derZitronensäure/Ethylenglycol-Lösung, Rühren der gemischten Lösungund Erhitzen der gerührten Lösung unter vollständigem Entfernen des Wassers daraus;
3) Ausführen einer schrittweisen Calcinierung des Materials von Schritt 2), aus dem das Wasser entfernt wurde; und
4) Einarbeiten von mindestens drei Metall-Vorstufen, einschließlich einer Eisen-Vorstufe und einer Kalium-Vorstufe, in den Calciumoxid-Träger durch gemeinsame Ausfällung oder Imprägnierung,
wobei derCalciumoxid-Träger eine spezifische Oberfläche von 5-50 m²/g aufweist, und der F-T-Katalysator100 Gewichtsteile eines Calciumoxid-Trägers; 10-70 Gewichtsteile Fe; 1-10 Gewichtsteile K und 1-10 Gewichtsteile von mindestens einem Metallelement, ausgewählt aus der Gruppe, bestehend aus Cu, Co und Mn,
umfasst.

2. Verfahren nach Anspruch 1, wobei die Calcium-VorstufeCalciumnitrat (Ca(NO₃)₂ 4H₂O) ist.

3. Verfahren nach Anspruch 1, wobei die Eisen-Vorstufe eine oder ein Gemisch von zwei oder mehreren, ausgewählt aus der Gruppe, bestehend aus Fe (II) und Fe (III)-Vorstufen, einschließlich Eisennitrat-Hydrat (Fe(NO₃)₃ 9H₂O), Eisenacetat (Fe(CO₂CH₃)₂), Eisenoxalat-Hydrat (Fe(C₂O₄)₃ 6H₂O), Eisenacetylacetonat (Fe(C₅H₇O₂)₃) und Eisenchlorid, ist.

4. Verfahren nach Anspruch 1, wobei die Metall-Vorstufenzusätzlich ein Metallsalz von einem, ausgewählt aus der Gruppe, bestehend aus Kupfer, Cobalt und Mangan, einschließen.

5. Verfahren nach Anspruch 1, das weiterhin einen Schritt des Calcinierens von so hergestelltem Katalysator bei einer Temperatur im Bereich von 300°C bis 700°C umfasst.

6. Verfahren nach Anspruch 1, wobei das Erhitzen in Schritt 2) bei einer Temperatur im Bereich von 100°C bis 150°C ausgeführt wird.

## Revendications

1. Procédé de préparation de catalyseurs F-T d'oxyde de fer, le procédé comprenant les étapes de :
1) dissolution d'acide citrique dans de l'éthylène glycol ;
2) addition d'une solution aqueuse d'un précurseur de calcium à la solution d'acide citrique/éthylène glycol, agitation de la solution mélangée, et chauffage de la solution agitée tout en éliminant complètement l'eau de cette dernière ;
3) réalisation d'une calcination par étapes du matériau de l'étape 2) duquel l'eau a été éliminée ; et
4) incorporation d'au moins trois précurseurs métalliques incluant un précurseur de fer et un précurseur de potassium dans le support d'oxyde de calcium par coprécipitation ou imprégnation,
dans lequel le support d'oxyde de calcium a une superficie spécifique de 5 à 50 m²/g, et le catalyseur F-T comprenant 100 parties en poids d'un support d'oxyde de calcium ; 10 à 70 parties en poids de Fe ; 1 à 10 parties en poids de K ; et 1 à 10 parties en poids d'au moins un élément métallique choisi dans le groupe consistant en Cu, Co et Mn.

2. Procédé selon la revendication 1, dans lequel le précurseur de calcium est le nitrate de calcium (Ca(NO₃)₂.4H₂O) .

3. Procédé selon la revendication 1, dans lequel le précurseur de fer est un ou un mélange de deux éléments ou plus, choisis dans le groupe consistant en des précurseurs de Fe (II) et Fe (III), incluant du nitrate de fer hydraté (Fe (NO₃)₃.9H₂O), de l'acétate de fer (Fe (CO₂CH₃)₂), de l'oxalate de fer hydraté (Fe (C₂O₄) ₃. 6H₂O), de l'acétylacétonate de fer (Fe(C₅H₇O₂)₃) et du chlorure de fer.

4. Procédé selon la revendication 1, dans lequel les précurseurs métalliques incluent en outre un sel de métal d'un élément choisi dans le groupe consistant en le cuivre, le cobalt et le manganèse.

5. Procédé selon la revendication 1, qui comprend en outre une étape de calcination du catalyseur ainsi préparé à une température allant de 300° C à 700° C.

6. Procédé selon la revendication 1, dans lequel le chauffage à l'étape 2) est réalisé à une température allant de 100° C à 150° C.
